# EUROPÄISCHE PATENTSCHRIFT

(11) **EP 0 534 444 B1**
(45) Veröffentlichungstag und Bekanntmachung des Hinweises auf die Patenterteilung: **27.03.1996**
(21) Anmeldenummer: 92116414.1
(22) Anmeldetag: 24.09.1992
(51) Int. Cl.: G01N 33/86, G01N 33/68, G01N 33/577, G01N 33/58, G01N 33/543, G01N 33/547

(54) **Verfahren zur Bestimmung von Fibrin**
Method for determining fibrin
Procédé pour déterminer de la fibrine

(30) Priorität: 25.09.1991 DE 4131953; 22.10.1991 DE 4134833
(43) Veröffentlichungstag der Anmeldung: 31.03.1993
(73) Patentinhaber: BOEHRINGER MANNHEIM GMBH, D-68298 Mannheim (DE)
(72) Erfinder: Dessauer, Andreas, Dr., W-8132 Tutzing (DE); Lill, Helmut, Dr., W-8121 Wielenbach (DE); Naser, Werner, Dr., W-8120 Weilheim (DE); Ofenloch-Hähnle, Beatus, Dr., W-8121 Haunshofen (DE)
(74) Vertreter: Huber, Bernhard, Dipl.-Chem.

(56) Entgegenhaltungen:
- EP-A- 0 152 612
- EP-A- 0 306 813
- EP-A- 0 356 964
- ARCHIVES OF BIOCHEMISTRY & BIOPHYSICS, Band 56, 1955, New York, NY (US); T.H. DONNELLY et al., Seiten 369-387/
- PROCEEDINGS OF THE NATL. ACADEMY OF SCIENCES USA, Band 82, Oktober 1985, Washington, DC (US); U. SCHEEFERS-BORCHEL et al., Seiten 7091-7095.
- CHEMICAL ABSTRACTS, vol. 93, no. 21, 24. November 1980, Columbus, OH (US); L.A. MOROZ, Seite 450, Nr. 201886n/
- THROMBOSIS RESEARCH, Band 27, Nr. 6, 15. September 1982, New York, NY (US); M. RANBY et al., Seiten 743-749/
- BLOOD, Band 47, Nr. 6, Juni 1976, New York, NY (US); R. LARGO et al., Seiten 991-1002/

## Beschreibung

Die vorliegende Erfindung betrifft ein Verfahren zur Behandlung von Fibrin enthaltenden Lösungen, durch das eine zuverlässigere Bestimmung von Fibrin in Körperflüssigkeiten, insbesondere eine verbesserte immunologische Fibrinbestimmung durch einen heterogenen Sandwich-Immunoassay unter Verwendung eines monoklonalen fibrinspezifischen Antikörpers ermöglicht wird.

Fibrin entsteht bei Aktivierung des Blutgerinnungssystems durch Einwirkung von aktivem Thrombin auf Fibrinogen. Das Fibrinogen-Molekül besteht aus zwei Paaren von α-, β- und γ-Ketten, die durch eine Vielzahl von Disulfidbrücken - speziell im Bereich der N-terminalen E-Domäne - miteinander verbunden sind. Durch Thrombin werden an den N-terminalen Enden des Fibrinogens die Fibrinopeptide A von den beiden α-Ketten und nachfolgend auch die beiden Fibrinopeptide B von den β-Ketten abgespalten. Die entstehenden Fibrinmonomere werden deshalb als Des-AA-Fibrin oder Fibrin I und Des-AABB-Fibrin oder Fibrin II bezeichnet. Diese Fibrinmonomere lagern sich dann aneinander und vernetzen zum Fibringerinnsel. Normalerweise sind lösliche Fibrinmonomere im Blut nicht vorhanden. Hingegen ist lösliches Fibrin bei verschiedenen Gerinnungsstörungen, insbesondere der Verbrauchs-Coagulopathie nachweisbar. Für die klinische Chemie ist es daher wichtig, beide Formen von Fibrin in Körperflüssigkeiten zuverlässig neben Fibrinogen nachweisen zu können.

Ein spezifischer Sandwich-Immunoassay zum Nachweis von Fibrin unter Verwendung eines fibrinspezifischen monoklonalen Antikörpers als Fängerantikörper ist beschrieben (Scheefers-Borchel et al., Proc.Natl.Acad.Sci. USA 82 (1985), 7091-7095). Der als Fängerantikörper verwendete fibrinspezifische monoklonale Antikörper wurde durch Immunisierung mit dem N-terminalen Hexapeptid der α-Kette von Fibrin erhalten. Dieses Peptid hat die Sequenz Gly-Pro-Arg-Val-Val-Glu. Der Nachweis einer Bindung von Fibrin an diesen Antikörper erfolgt im obigen Testverfahren mit einem polyklonalen peroxidasemarkierten <Fibrinogen>-Antikörper.

Es wurde nun festgestellt, daß bei der Bestimmung von Fibrin in Körperflüssigkeiten nach dieser Methode relativ häufig unplausibel niedrige Fibrinwerte erhalten werden bzw. Fibrin überhaupt nicht nachgewiesen werden kann, obwohl ein anderes Fibrinbestimmungsverfahren eindeutig das Vorhandensein von Fibrin anzeigte.

Ein anderes Problem ist, daß mit der Methode nach dem Stand der Technik z.T auch Werte in Proben gemessen werden, die kein Fibrin enthalten, wie z.B. Normalplasmen. Das ist störend, da dadurch die Diskriminierung zwischen Null Fibrin und sehr kleinen Fibrin-Werten (ca. 1 µg/ml) erschwert wird. Besonders gravierend wirkt sich das offensichtlich in automatisierten Testsystemen aus. Der hohe Leerwert in Normalplasmen resultiert daraus, daß adsorptiv an die Gefäßwand gebundenes Fibrinogen vom POD-markierten Antikörper detektiert wird, da dieser nicht spezifisch für Fibrin ist.

Versucht man zur Beseitigung des hohen Probenleerwerts den gleichen fibrinspezifischen Antikörper sowohl als Fängerantikörper als auch als POD-markierten Antikörper zu verwenden, dann stellt man fest, daß dies nicht möglich ist. Mit dieser Testvariante werden nur "künstliche" Fibrinproben (Fibrinstandards), d.h. Plasmen, die mit Fibrinlösungen (z.B. Fibrin, gelöst in NaBr-Lösung, 1 mol/l) versetzt wurden, detektiert. Proben von Patienten dagegen, die erhöhte Fibrinwerte aufweisen, werden nicht als pathologisch erkannt. D.h. also, in dem Verfahren nach dem Stand der Technik ist es nicht möglich, den gleichen Fibrin-spezifischen Antikörper in einem Sandwich-Assay zu verwenden.

Es ist zwar bekannt, daß Bromid und Harnstoff in hohen Konzentrationen durchaus in der Lage sind, beispielsweise Fibrinclots aufzulösen (siehe z.B. Rånby et al., Thrombosis Research 27 (1982), 743-749; Perlick und Bergmann, Gerinnungslaboratorium in Klinik und Praxis, Georg Thieme Verlag Leipzig 1971, S. 339; Donnelly et al., Arch.Biochem.Biophys. 56 (1955), 369-387). Nach Verdünnen von Fibrinlösungen, die auf diese Weise behandelt worden waren, konnte jedoch keine Verbesserung der Fibrinbestimmung festgestellt werden.

Aufgabe der vorliegenden Erfindung war somit die Bereitstellung eines Verfahrens, bei dem die Nachteile des Standes der Technik mindestens teilweise beseitigt sind, insbesondere die Zuverlässigkeit und Sensitivität der Fibrinbestimmung verbessert wird.

Die erfindungsgemäße Aufgabe wird gelöst durch ein Verfahren zur Behandlung einer Fibrin enthaltenden Lösung, welches dadurch gekennzeichnet ist, daß man die Lösung mit Rhodanid-, Jodid-, Magnesium- oder/und Guanidiniumionen versetzt und inkubiert. Die Konzentration der oben genannten Ionen wird dabei günstigerweise ausreichend hoch gewählt, daß die Fibrinmoleküle in der Probelösung irreversibel in eine der immunologischen Bestimmung zugängliche Form, vermutlich durch Auflösung von Komplexen oder Aggregaten, gebracht werden.

Durch die erfindungsgemäße Behandlung von Fibrin enthaltenden Lösungen gelingt überraschenderweise eine erheblich zuverlässigere Bestimmung von Fibrin in Körperflüssigkeiten. Ein weiterer Gegenstand der vorliegenden Erfindung ist daher eine Methode zur Bestimmung von Fibrin in Körperflüssigkeiten, die dadurch gekennzeichnet ist, daß man die Fibrin enthaltende Probelösung vor Durchführung der Bestimmung mit Rhodanid-, Jodid-, Magnesium- oder/und Guanidiniumionen versetzt, inkubiert, gegebenenfalls nach der Inkubation verdünnt und anschließend den Fibringehalt der Probelösung ermittelt.

Überraschenderweise wurde festgestellt, daß bei Behandlung von Fibrin enthaltenden Probelösungen mit Rhodanid-, Jodid-, Magnesium- oder/und Guanidiniumionen die Zugänglichkeit des Fibrins erheblich verbessert wird, so daß eine starke Sensitivitätssteigerung des Fibrinnachweises erzielt werden kann. Besonders überraschend ist, daß nur wenige, ganz bestimmte Substanzen hierfür geeignet sind. Beispielsweise hat sich gezeigt, daß Chlorid, Bromid, Arginin und Harnstoff nicht geeignet sind.

Bei Durchführung der erfindungsgemäßen Bestimmungsmethode findet man Fibrin auch in Plasmaproben, bei denen die bekannte Methode nach Scheefers-Borchel et al. versagt, wo aber mit einer dritten Methode pathologische Fibrinwerte gemessen wurden. Diese dritte Methode ist der FM-Test der Boehringer Mannheim GmbH. Dabei handelt es sich um einen Agglutinationstest, der mit fibrinbeschichteten Eythrozyten arbeitet (Largo et al., Blood 47 (1976), 991). Bei Anwesenheit von Fibrin in einer Probe klumpen die Erythrozyten zusammen und ermöglichen so einen halbquantitativen Fibrinnachweis. Die Sensitivität dieses Tests ist mit ≧ 10 µg/ml relativ gering, dennoch erhält man bei diesem Test häufig positive Ergebnisse, während der immunologische Test nach Scheefers-Borchel kein Fibrin oder aber Menge von << 10 µg/ml anzeigt.

Die Dauer und Temperatur der Inkubation bei dem erfindungsgemäßen Verfahren sind im allgemeinen nicht besonders kritisch. Günstigerweise wird jedoch die Lösung für 1 bis 60 Minuten, vorzugsweise 15 bis 30 Minuten nach Zugabe der ionischen Substanzen bei einer Temperatur von 10 bis 40°C, vorzugsweise 15 bis 30°C durchgeführt. Bei geringeren Inkubationstemperaturen verlängert sich im allgemeinen dementsprechend die Inkubationsdauer.

Wird die Fibrin enthaltende Lösung mit Rhodanidionen inkubiert, so wird das Rhodanid der Probelösung vorzugsweise in Form von Alkalimetall-, Magnesium- oder Ammoniumrhodanidlösungen zugesetzt. Dabei sollte die Konzentration von Rhodanidionen in der Probelösung günstigerweise 0,5 mol/l übersteigen und liegt vorzugsweise zwischen 0,5 und 8,5 mol/l und besonders bevorzugt zwischen 0,7 und 7 mol/l, wobei auch bei großen Rhodanidkonzentrationen kein Nachweis von Fibrinogen erfolgt, d.h. es treten keine erhöhten Fibrinwerte bei der Bestimmung von Normalplasma auf. Die Verwendung von Rhodanidionen ist besonders bevorzugt bei der Durchführung des erfindungsgemäßen Verfahrens.

Inkubiert man die Fibrin enthaltende Probelösung mit Jodidionen, so werden diese vorzugsweise in Form von Alkalimetall-, Magnesium- oder Ammoniumjodidlösungen zugesetzt. Günstigerweise sollte die Konzentration von Jodidionen in der Probelösung 1 mol/l übersteigen und liegt vorzugsweise zwischen 2 und 8 mol/l und besonders bevorzugt zwischen 2 und 4 mol/l. Weiterhin wurde festgestellt, daß bei Inkubation der Lösung mit einer Kombination von Rhodanid- und Jodidionen ein synergistischer Effekt beobachtet wird, d.h. die Kombination beider Ionen geht über die Summe der Einzelwirkungen hinaus. Bei einer derartigen Ausführungsform des erfindungsgemäßen Verfahrens beträgt die Konzentration von Rhodanidionen vorzugsweise zwischen 0,2 und 2 mol/l und die Konzentration von Jodidionen zwischen 2 und 8 mol/l. Besonders bevorzugt beträgt die Konzentration von Rhodanidionen zwischen 0,5 und 1,5 mol/l und die Konzentration von Jodidionen zwischen 3 und 4 mol/l. Ganz besonders bevorzugt ist der Bereich von 0,5 bis 1,0 mol/l für Rhodanidionen.

Bei Inkubation der Probelösung mit Guanidiniumionen werden diese vorzugsweise in Form von Guanidiniumhydrohalogenid-, besonders bevorzugt Guanidiniumhydrochloridlösungen zugeführt. Die Konzentration von Guanidiniumionen in der Lösung sollte 1 mol/l übersteigen und liegt vorzugsweise zwischen 1 und 4 mol/l.

Bei Inkubation der Fibrin enthaltenden Probelösung mit Magnesiumionen werden diese vorzugsweise in Form von löslichen Magnesiumsalzen, z.B. Magnesiumhalogeniden oder Magnesiumnitrat, zugesetzt. Die Konzentration der Magnesiumionen in der Probelösung sollte günstigerweise mindestens 0,5 mol/l, bevorzugt zwischen 1 und 3 mol/l und besonders bevorzugt zwischen 1 und 2 mol/l betragen.

Durch die erfindungsgemäße Behandlung einer Fibrin enthaltenden Lösung wird bei einer anschließenden Bestimmung von Fibrin eine erhöhte Sensitivität erreicht. Nach dem erfindungsgemäßen Inkubationsschritt und vor der eigentlichen Bestimmung hat sich aufgrund der bei der Inkubation herrschenden hohen Salzkonzentrationen eine Verdünnung der Probelösung im allgemeinen als erforderlich erwiesen. Dabei wird die Probelösung vorzugsweise mit einem geeigneten Puffer im Verhältnis von 1:5 bis 1:500 von Probelösung zu Puffer, besonders bevorzugt von 1:10 bis 1:200 verdünnt. Es ist zu beachten, daß die erfindungsgemäß geeigneten Substanzen nicht einfach nur in verdünnter Lösung zur Ablösung des Fibrins aus Komplexen geeignet sind, sondern daß es im allgemeinen zur Ablösung notwendig ist, die Substanzen in hoher Konzentration auf die Probe einwirken zu lassen, um die Ablösung des Fibrins zu bewirken. Erst anschließend kann verdünnt werden.

Dabei wurde festgestellt, daß bessere Ergebnisse bei der Fibrinbestimmung erhalten werden, wenn der pH-Wert der Probelösung nach dem erfindungsgemäßen Inkubationsschritt auf einen Bereich von 5,5 bis 7,5, vorzugsweise auf einen Bereich von 5,8 bis 6,2 eingestellt wird.

Aufgrund der Probenverdünnung nach der Inkubation mit einem geeigneten Puffer, wobei der pH-Wert der Probelösung besonders bevorzugt auf pH 6,0 eingestellt wird, werden Fibrin I und Fibrin II gleich gut detektiert. Als geeigneter Puffer hat sich beispielsweise ein 0,1 mol/l Phosphatpuffer pH 6,0 erwiesen. Es wurden jedoch auch mit einem Acetat- und einem Citratpuffer gute Ergebnisse erhalten. Die Wirkung des Puffers beruht daher nicht auf einem spezifischen Effekt der Phosphationen, sondern es liegt ein Effekt des pH-Werts vor.

Die Einstellung der Probelösung auf einen pH-Wert zwischen 5,5 und 6,5 kann im Prinzip auch unter Verwendung einer laborüblichen Säure erreicht werden, es ist jedoch praktikabler, als Verdünnungsreagenz einen Puffer zu verwenden, dessen Pufferkapazität ausreichend stark sein muß, um nach Mischung mit dem Plasma einen pH-Wert von 5,5 bis 6,5, besonders bevorzugt von 6,0 zu gewährleisten. Bei pH-Werten von < 5,5 werden auch in Plasma ohne Fibrin (z.B. Normalplasma) bereits deutlich erhöhte Werte, d.h. falsch positive Werte, gefunden.

Der letzte Schritt der erfindungsgemäßen Methode zur Bestimmung von Fibrin in Körperflüssigkeiten umfaßt die Ermittlung des Fibringehalts der Probelösung. Dieser Schritt erfolgt vorzugsweise durch immunologische Verfahren, insbesondere unter Verwendung von mindestens einem fibrinspezifischen monoklonalen Antikörper.

Bei einer besonders bevorzugten Ausführungsform wird dabei der Fibringehalt der Probelösung durch einen heterogenen Sandwich-Assay ermittelt, wobei man die Probelösung mit einem immobilisierten oder immobilisierbaren fibrinspezifischen ersten Antikörper und einem markierten zweiten Antikörper, der an ein Konjugat von Fibrin und dem ersten Antikörper binden kann, in Kontakt bringt und die Markierung auf an sich bekannte Weise bestimmt.

Der erste Antikörper ist immobilisiert bzw. immobilisierbar, d.h. er liegt an eine Festphase gebunden vor oder er ist so modifiziert, daß er an eine Festphase bindefähig ist. Beispiele für immobilisierte Antikörper sind solche, die nach dem Fachmann bekannten Methoden an die Oberfläche einer Festphase, z.B. einen Plastikträger, fixiert sind. Eine derartige Fixierung kann beispielsweise durch nicht-kovalente Adsorption oder durch kovalente Kopplung des Antikörpers mit reaktiven Gruppen oder/und Spacermolekülen auf der Oberfläche der Festphase erfolgen. Ein Beispiel für einen immobilisierbaren Antikörper ist ein biotinylierter Antikörper, der an eine mit Streptavidin beschichtete Festphase bindefähig ist. Die Herstellung von biotinylierten Antikörpern erfolgt üblicherweise so, daß ein reaktives Biotinderivat mit SH-Gruppen des Antikörpers zur Bildung des biotinylierten Antikörpers umgesetzt wird. Anstelle eines Antikörpers können dabei auch Antikörperfragmente wie etwa F(ab)₂, Fab oder Fab'-Fragmente eingesetzt werden (F. Fieber, Biotinylierung monoklonaler Antikörper in: Monoklonale Antikörper/Herstellung und Charakterisierung, Springer-Verlag Berlin, Heidelberg, New York (1990) 299-302).

Der erste Antikörper muß ein fibrinspezifischer Antikörper sein, d.h. er darf keine nennenswerte Reaktivität gegenüber Fibrinogenmolekülen besitzen. Die Herstellung von fibirinspezifischen monoklonalen Antikörpern kann durch Immunisierung von Versuchstieren mit dem Hexapeptid Gly-Pro-Arg-Val-ValGlu gemäß dem Stand der Technik erhalten werden. Ein Beispiel für einen fibrinspezifischen monoklonalen Antikörper, der beim erfindungsgemäßen Verfahren eingesetzt werden kann, ist der Antikörper 2B5 (ECACC 89112802), der durch Immunisierung von Versuchstieren mit dem Heptapeptid Gly-Pro-Arg-Val-ValGlu-Arg erhalten wurde. Zur Immunisierung wird das Peptid in bekannter Weise gebunden an Trägerproteine wie KLH (Keyhole Limpet Hemocyanine) eingesetzt (Proc.Natl.Acad.Sci. USA 78 (1981) 3404).

Der zweite Antikörper ist ein markierter Antikörper, der mit dem Konjugat aus erstem Antikörper und Fibrin reagieren kann. Die Markierung dieses Antikörpers kann beispielsweise eine enzymatische, fluoreszierende, lumineszierende, radioaktive oder NMR-aktive Gruppe sein. Beispiele für derartige Markierungsgruppen sind dem Fachmann bekannt. Vorzugsweise trägt der Antikörper eine enzymatische Markierungsgruppe, z.B. Peroxidase (POD) oder alkalische Phosphatase. Die POD-Markierung kann beispielsweise nach Wilson und Nakane, Recent developments in the periodate method of conjugating horseradish peroxidase (HRPO) to antibodies, in: Immunofluorescence and related staining techniques, (W. Knapp, K. Holubar, and G. Wick, eds.), Elsevier/North-Holland Biomedical Press, Seiten 215-224, erfolgen.

Der zweite Antikörper kann der gleiche Antikörper wie der erste Antikörper sein, d.h. er kann eine gleiche Bindungsstelle besitzen. Andererseits kann der zweite Antikörper vom ersten Antikörper verschieden sein. Beispielsweise sind als zweiter Antikörper auch solche Antikörper geeignet, die neben Fibrin auch noch Fibrinogen erkennen. Ein solcher Antikörper ist z.B. der Antikörper 1.156.317 (ECACC Nr. 89060902). Ferner kann der zweite Antikörper auch ein polyklonaler Antikörper oder ein Antikörperfragment sein.

Ein weiteres immunologisches Testverfahren, für das sich die erfindungsgemäße Methode der Probenvorbereitung eignet, ist ein Test unter Verwendung von Latex. Bei diesem Latextest lagern sich Latexpartikel, welche an der Oberfläche Antikörper gegen Fibrin tragen, an Fibrin (welches zwei gleiche Epitope trägt) an, wobei eine Aggregation erfolgt und die Trübung gemessen werden kann. Geeignete Testprinzipen sind beispielsweise in J. Clin. Immunoassay 13 (1990), 127-131 und EP-A 0356 964 beschrieben.

Schließlich ist noch festzustellen, daß die erfindungsgemäße Inkubation einer Fibrin enthaltenden Lösung mit Rhodanid-, Jodid, Magnesium- oder Guanidiniumionen in einem gewissen Ausmaß von der anschließenden Bestimmungsmethode des Fibringehalts in der Probelösung abhängen kann. So sind bei automatisierten Tests unter Verwendung des gleichen fibrinspezifischen Antikörpers als immobilisiertem und als markiertem Antikörper und bei Einsatz der Streptavidin-Biotin-Technologie Rhodanidkonzentrationen von 1,5 bis 6,5 mol/l in der Probenlösung gut geeignet. Besonders bevorzugt ist eine Rhodanidkonzentration von 4,0 mol/l. Bei Verwendung eines fibrinspezifischen immobilisierten Antikörpers und eines zweiten unspezifisch markierten Antikörpers ohne Streptavidin/ Biotin ist eine Konzentrationsbereich von 0,7 bis 1,5 mol/l Rhodanid in der Probenlösung gut geeignet. Besonders bevorzugt wird etwa 1,2 mol/l Rhodanid bei der Inkubation der Probe eingesetzt.

Ein weiterer Gegenstand der Erfindung ist auch ein Reagenzienkit, der Rhodanid-, Jodid-, Magnesium- oder/und Guanidiniumsalze in fester oder/und gelöster Form sowie Reagenzien für die Fibrinbestimmung enthält.

Die Antikörper 2B5 und 1.156.317 wurden gemäß den Bestimmungen des Budapester Vertrags mit den Hinterlegungsnummern ECACC 89112802 bzw. ECACC 89060902 bei ECACC, Porton Down, GB hinterlegt.

Die Erfindung soll weiterhin durch die folgenden Beispiele verdeutlicht werden.

### Beispiel 1

Bestimmung von Fibrin mit einem immobilisierten fibrinspezifischen Antikörper und einem unspezifischen, POD-markierten Antikörper.

### a) Allgemeines:

Die Antikörper-POD-Konjugate wurden, wenn nicht anders angegeben mit Konjugatpuffer gelöst und auf eine Konzentration von 80 - 120 mU/ml Peroxidase-Aktivität eingestellt. Es werden nicht komplette IgGs eingesetzt, sondern die Fab-Fragmente.

### b) Testdurchführung (allgemein)

Im ersten Inkubationsschritt wird ein spezifischer Antikörper an einen Plastikträger (NUNC-Mikrotiterplatten) fixiert. Um unspezifische Bindungen zu verhindern, werden die nicht abgesättigten Bindungsstellen der Festphase durch ein Nachbelade-Medium abgesättigt. Im zweiten Inkubationsschritt bindet dieser Antikörper das Fibrin aus der Probe (1.Immunreaktion). Das Fibrin besitzt hierfür zwei antigene Determinanten. Es werden in der anschließenden Immunreaktion mit POD-markierten Fibrin-Antikörpern, Sandwich-Komplexe gebildet (2. Immunreaktion). Die Menge der gebildeten Sandwich-Komplexe stellt ein Maß für die Konzentration von Fibrin in der Probe dar. Im nachfolgenden Waschschritt (bound-free separation) wird das nichtgebundene POD-Konjugat entfernt. Nach Zugabe von Chromogen (ABTS^{®}) wird die an der Röhrchenwand gebundene POD-Aktivität photometrisch bestimmt. Zur Auswertung wird zu jeder Meßserie eine Bezugskurve erstellt. Hierzu wird eine Fibrin-Standardlösung mit Probenverdünnungs-Lösung verdünnt und entsprechend Beispiel 1d, Teil a vorbehandelt.

### c) Reagenzien

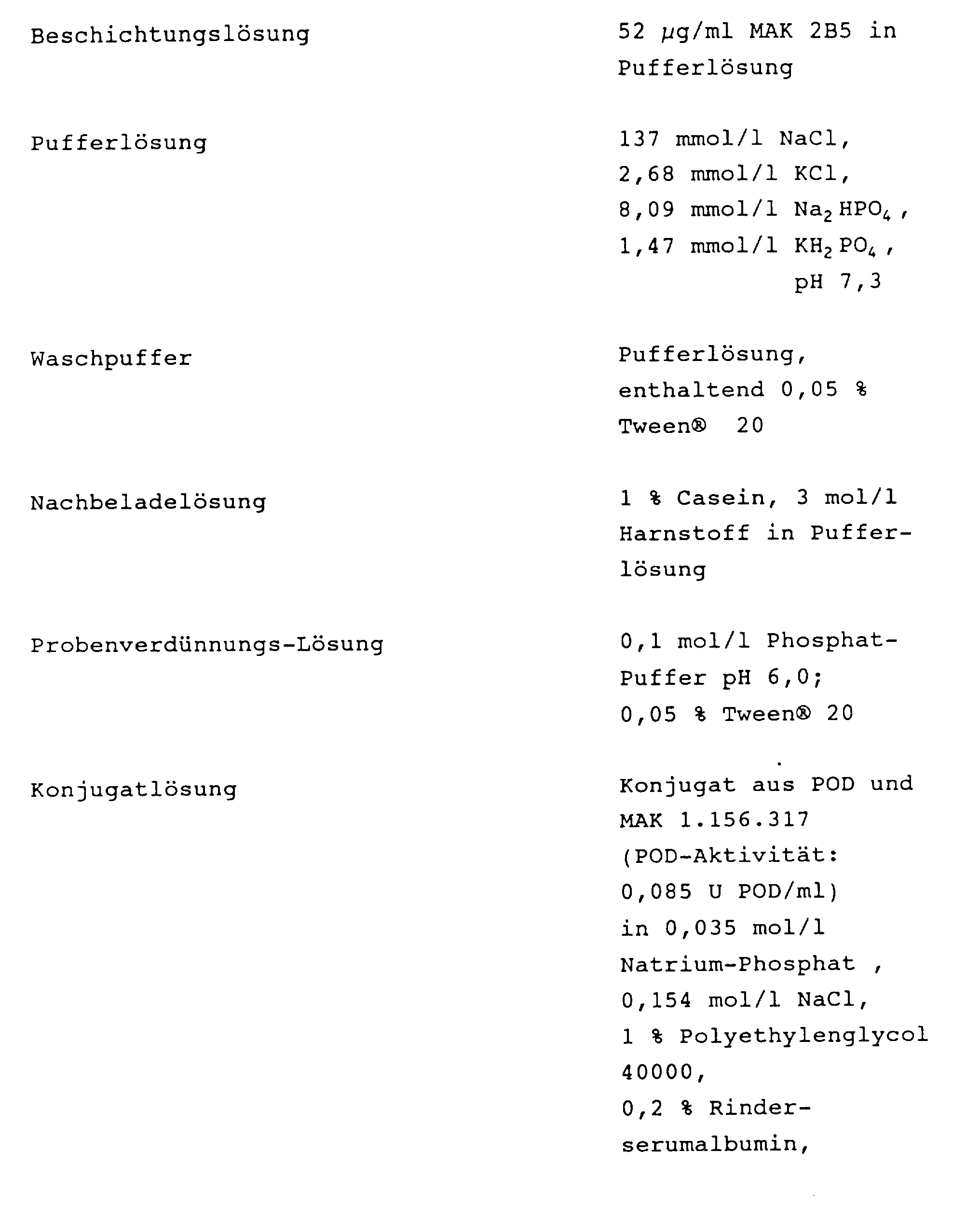

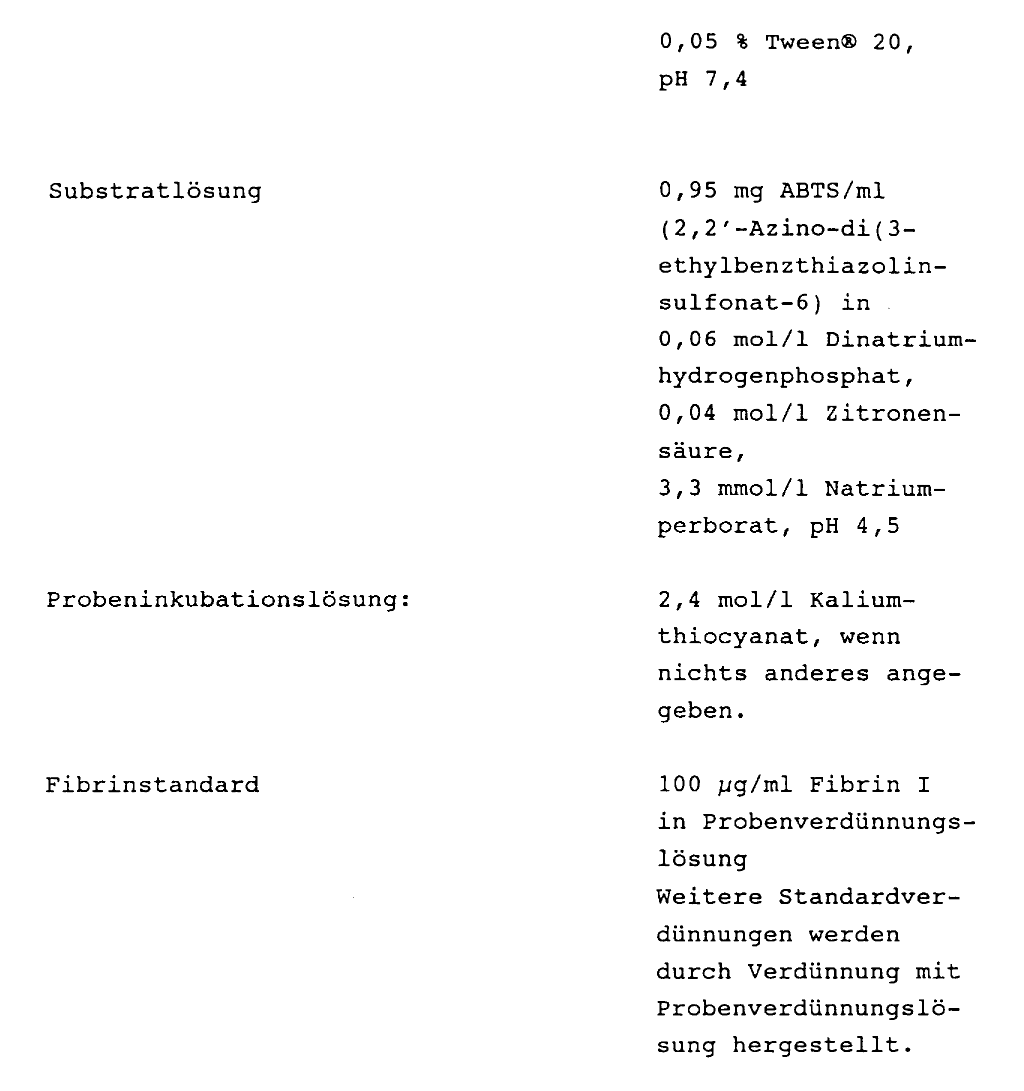

### d) Probe

### Probengewinnung (Citratplasma)

9 Teile frisch entnommenes Blut werden mit 1 Teil Antikoagulans gemischt und anschließend 10 Minuten bei 2000 g zentrifugiert (bei normalen Laborzentrifugen ca.3000 U/Min.). Der Überstand wird abpipettiert.

Dann wird, wie beschrieben, vorgegangen, d.h.;

### Probenvorbereitung

### a) Variante ohne die genannten Salze:

1 Teil Probe wird mit 100 Teilen Probenverdünnungslösung gemischt. Diese Mischung wird im weiteren als Probenlösung bezeichnet.

### b) Variante mit den genannten Salzen:

1 Teil Probe wird mit 1 Teil Probeninkubationslösung (z.B. KSCN, 2,4 mol/l) gemischt und bei Raumtemperatur 30 Minuten lang inkubiert. Diese Mischung wird im weiteren als Probenlösung bezeichnet.

Die Probeninkubationslösung hat somit die doppelte Konzentration an Rhodanid-, Jodid-, Magnesium- und/oder Guanidiniumionen als die Probenlösung.

Dann wird ein Teil dieser Lösung mit 50 Teilen Probenverdünnunglösung (Phosphatpuffer, pH 6,0) verdünnt (bezogen auf die 1+1-Mischung)

e) 0,1 ml Beschichtungslösung werden pro well in eine Mikrotiterplatte gegeben. Es wird 30 min bei Raumtemperatur geschüttelt, entleert und 0,15 ml Nachbeladelösung zugegeben. Nach 15 min Schütteln bei Raumtemperatur wird zweimal mit je 0,3 ml Waschpuffer gewaschen.

0,1 ml Probe pro well, die nach Beispiel 1 (d) vorbereitet wurde, wird zugegeben. Es wird 30 min bei Raumtemmperatur geschüttelt und dreimal mit je 0,3 ml Waschpuffer gewaschen. Es werden 0,1 ml POD-markierter Antikörper zugegeben, 15 min bei Raumtemperatur geschüttelt und viermal mit je 0,3 ml Waschpuffer gewaschen. Es wird 0,1 ml Substratlösung zugegeben, 15 min bei Raumtemperatur geschüttelt und die Extinktion bei 410 nm bestimmt.

| | |
|---|---|
| Wellenlänge: | 410 nm |
| Referenzwellenlänge: | 490 nm |
| Messung: gegen Reagenzien-Leerwert | |

### f) Erstellen der Eichkurve

Zur Kalibration wird eine Fibrin-Standardlösung (100 µg/ml) eingesetzt.

### Mischtabelle:

| | | | | | | |
|---|---|---|---|---|---|---|
| | Konzentrationen der Fibrinstandard-Verdünnungen | | | | | |
| µg/ml Fibrin | 0 | 10 | 25 | 50 | 75 | 100 |
| Fibrin-Standard 100 µg/ml | - | 10 µl | 25 µl | 50 µl | 75 µl | 100 µl |
| Probenverdünnungs-Lösung | 100 µl | 90 µl | 75 µl | 50 µl | 25 µl | - |

Alle Standard-Verdünnungen müssen vor dem Einsatz in den Test wie die Proben vorbehandelt bzw. verdünnt werden.

### g) Fibrinbestimmung nach erfindungsgemäßer Probenvorbereitung

Durch Zugabe von Rhodanid-Ionen zur Probenlösung wird eine starke Sensitivitätssteigerung des Fibrin-Nachweises erzielt (Tabelle 1).

Humanes Citratplasma wurde mit Spuren von Thrombin (50 mU Thrombin pro 10 ml Plasma) und nach 10 min mit 2,0 USP-Einheiten Heparin pro ml Plasma versetzt, um die Thrombinaktivität zu hemmen. Zur Ermittlung eines Anfangswerts wurde Thrombin in Heparin-haltiges Plasma pipettiert (0 min-Wert). Die Proben wurden dann analog Beispiel 1d vorbehandelt.

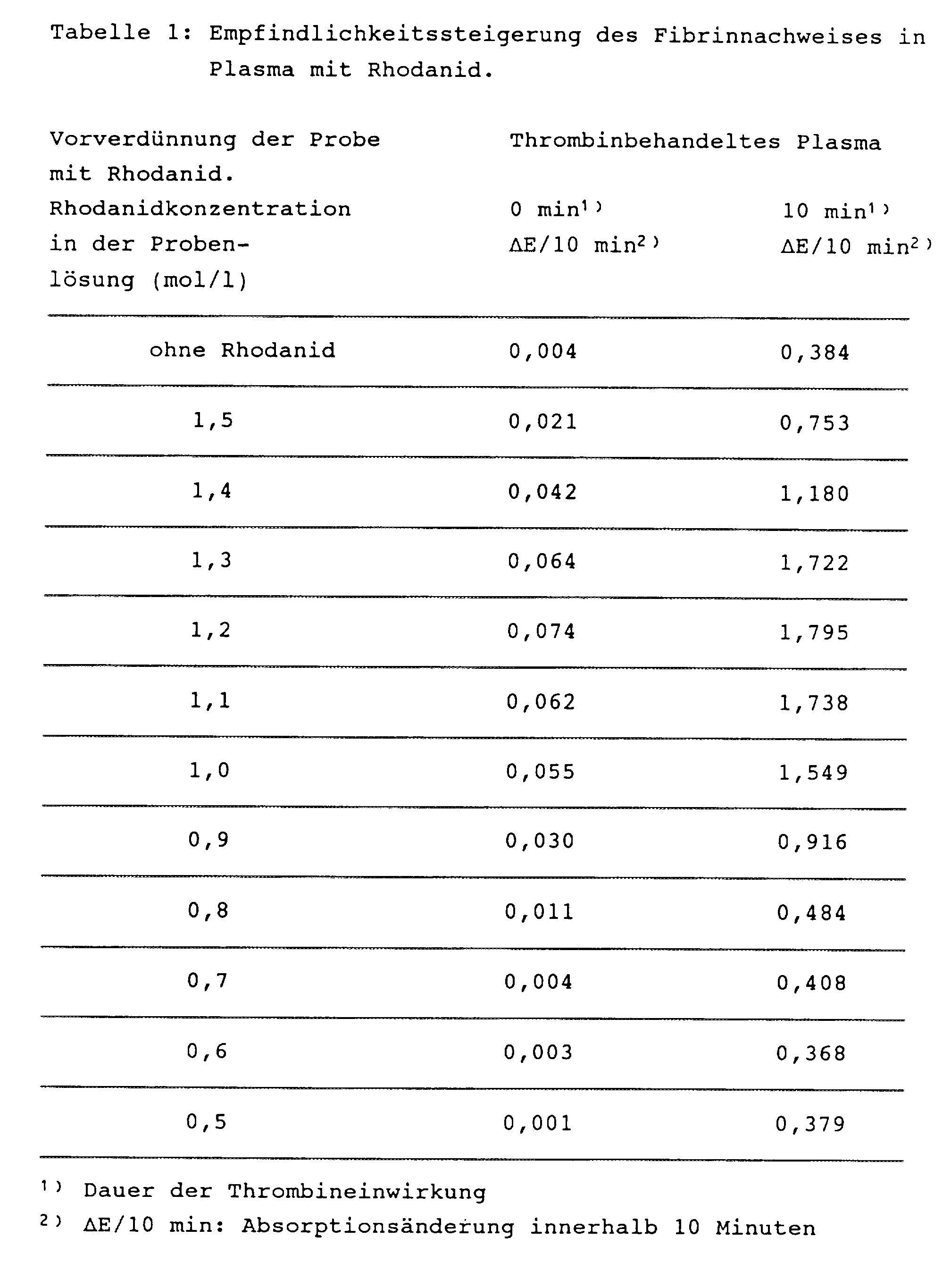

Ergebnis: Die Inkubation der Probe mi Rhodanid bringt in einem wieten Konzentrationsbereich eine Empfindlichkeitssteigerung des Fibrinnachweises in Plasma.

Auch die Probenvorbehandlung mit Guanidiniumhydrochlorid verbessert die Empfindlichkeit des Fibrinnachweises (Tabelle 2).

Probenvorbereitung und Durchführung der Bestimmung erfolgte analog zu den Versuchen mit Rhodanidzusatz.

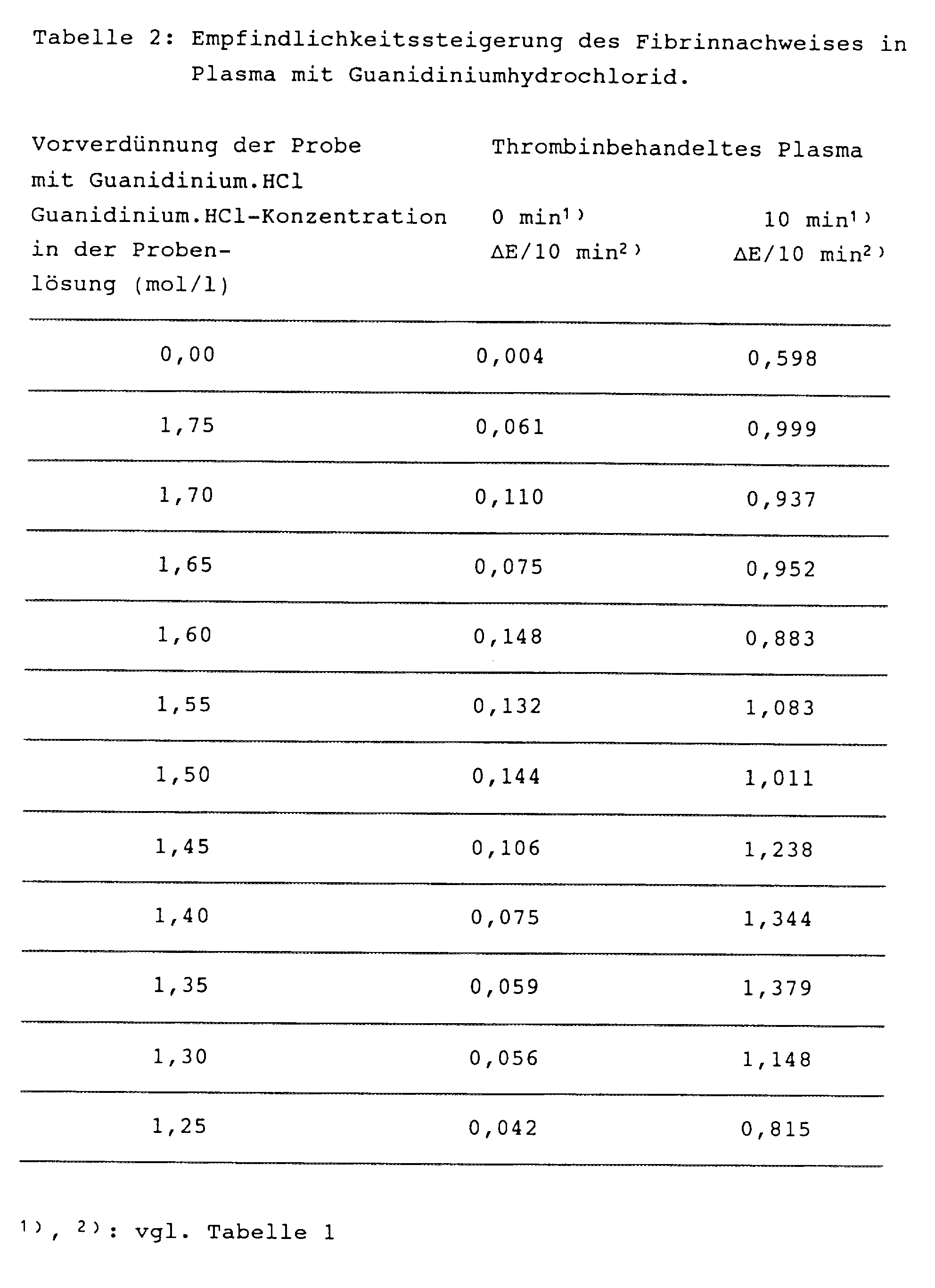

In einem weiteren Experiment wurde einer gesunden Person Blut abgenommen, ohne daß zunächst ein Antikoagulans zugesetzt wurde. Zu den in Tabelle 3 angegebenen Zeiten wurden Aliquots des abgenommenen Blutes mit einem Antikoagulans versetzt (9 Teile Blut + 1 Teil Antikoagulans, bestehend aus Tri-Natriumcitrat, 0,11 Mol/L; Heparin, 200 IE/ml; ε-Aminocapronsäure, 0,02 mol/l; Aprotinin, 0,33 mg/100 ml). Anschließend wurde zentrifugiert und das gewonnene Plasma für den Fibrin-Test verwendet. Das Antikoagulans verhindert die Aktivierung des Gerinnungssystems. Bereits vorhandene Gerinnungsproteasen, insbesondere Thrombin werden durch Heparin inaktiviert. Es kann also nach Zugabe kein weiteres Fibrin mehr gebildet werden und die Plasmaprobe wird in ihrem jeweiligen Aktivierungszustand konserviert. ε-Aminocapronsäure und Aprotinin verhindern eine Aktivierung des fibrinolytischen Systems und wirken somit einem Abbau des zum jeweiligen Zeitpunkt bereits gebildeten Fibrins entgegen.

### (A) Verdünnung der Probe nach Beispiel 1d, Teil a.

### (B) Vorbehandlung der Probe mit Rhodanid analog Beispiel 1d, Teil b (2,4 mol/l Kaliumrhodanid in der Probeninkubationslösung , 15 min Inkubation bei Raumtemperatur).

**Tabelle 3**

| Aktivierung von Blut durch Stehenlassen ohne Antikoagulanz | | |
|---|---|---|
| Zeitpunkt der Zugabe | Verdünnung der Plasmaproben nach Variante des Koagulans | |
| (min) | (A) E/10 min | (B) E/10 min |
| 0 | 0,014 | 0,097 |
| 5 | 0,011 | 0,105 |
| 10 | 0,020 | 0,112 |
| 17,5 | 0,057 | 0,167 |
| 20 | 0,222 | 0,609 |
| 22,5 | 0,352 | 1,015 |

### Ergebnis: Rhodanidzusatz der Probe bewirkt eine deutliche Sensitivitätssteigerung des Testsignals.

### Beispiel 2

Immunologische Bestimmung von Fibrin mit immobilisiertem, biotinyliertem Fibrin-spezifischen Antikörper und Peroxidase-(POD)-markiertem Antikörper

Es wird ein Fibrin-spezifischer monoklonaler Antikörper, in biotinylierter Form verwendet; - zur Verwendung kommen Streptavidin-beschichtete Reaktionsgefäße (Herstellung nach EP-A 0 269 092)

### Reagenzien

Biotinylierter Antikörper, Arbeitslösung: 1,3 µg/ml Konjugat aus Antikörpern 2B5 und Biotin in Kaliumdihydrogenphosphat, 0,085 mol/l, Dikaliumhydrogenphosphat, 0,015 mol/l Rinderserumalbumin, 0,5 % Tween® 20 0,05 %.

### Waschlösung: Natriumchlorid 4,3 mmol/l.

POD-Konjugatlösung: Konjugat aus Antikörper 2B5 und POD [POD Aktivität 0,139 U/ml) in Natriumphosphat 0,035 mol/l, NaCl 0,154 mol/l, Polyäthylenglycol 40000, 1 %, Rinderserum-Albumin, 0,2 % Tween®, 20 0,05 %, pH 7,4.

### Substratlösung (ABTS®):

0,95 mg ABTS®/ml in Dinatriumhydrogenphosphat 0,06 mol/l, Citronensäure 0,04 mol/l, Natriumperborat 3,3 mmol/l, pH 4,5.

### Durchführung der Bestimmung:

20 µl Probe werden nach Beispiel 1d vorbehandelt (Konzentrationen von Rhodanid, Jodid oder Guanidiniumhydrochlorid vgl. Tabellen). Es wird 1 ml Arbeitslösung des biotinylierten Antikörpers zugegeben, 30 min bei Raumtemperatur inkubiert und die entleerten Reaktionsgefäße zweimal mit Waschlösung gewaschen.

Es wird 1 ml POD-Konjugatlösung zugegeben, 30 min bei Raumtemperatur inkubiert, und die entleerten Reaktionsgefäße zweimal mit Waschlösung gewaschen.

Es wird 1 ml Substratlösung zugegeben, 30 min bei Raumtemperatur inkubiert und die gebildete Farbe bei 405 nm bestimmt.

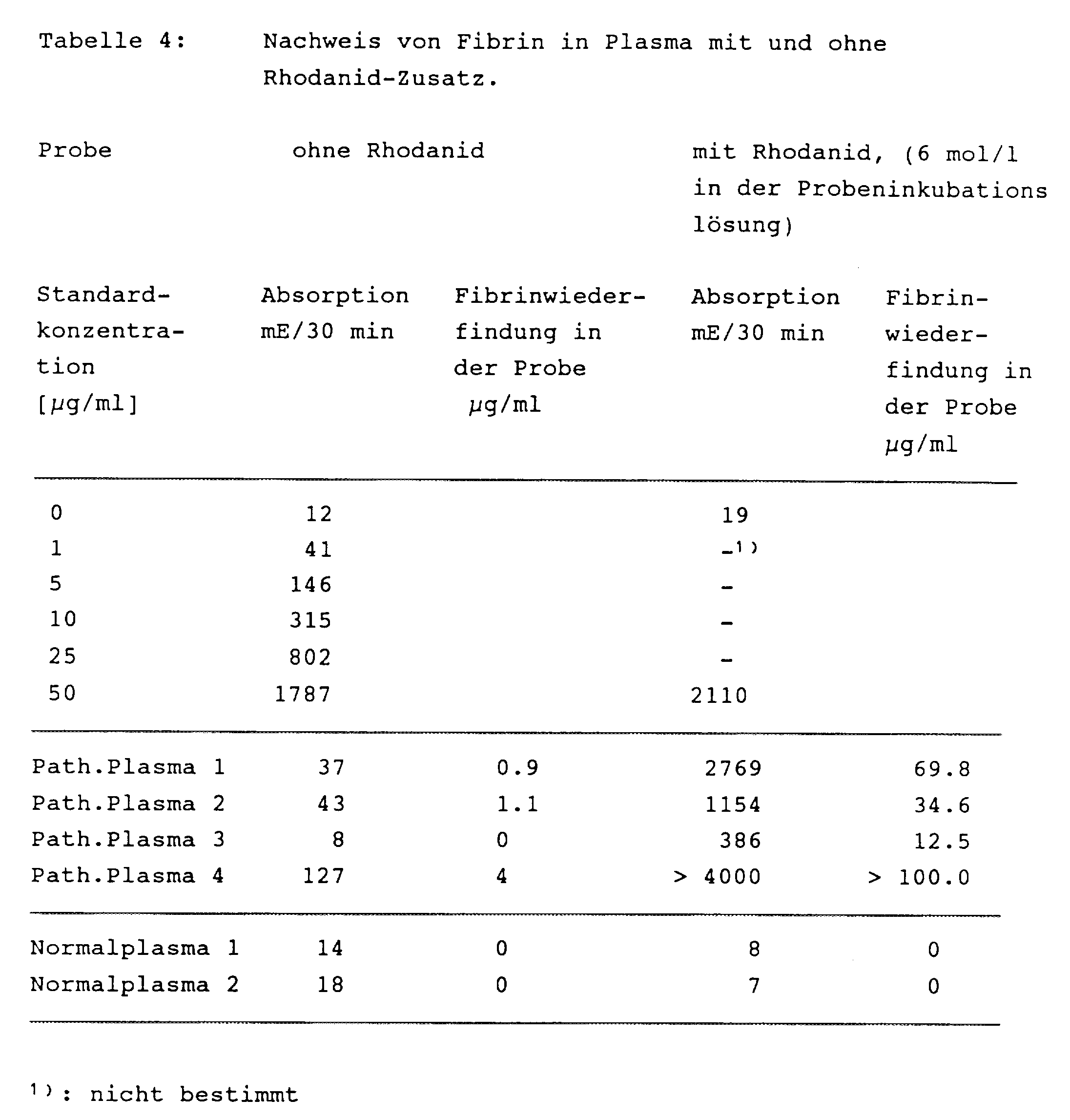

Ohne Zusatz von Rhodanid wird Fibrin nur in Fibrin-Standards, also in Proben, die durch Aufstocken von Normalplasma mit Fibrinlösungen in NaBr-Lösung, 1 mol/l erhalten wurden, erkannt.

Bei Verwendung von Rhodanid zur Probenvorbereitung wird dagegen auch Fibrin in den Patientenproben erkannt. Auch der Probenleerwert (Normalplasma) ist bei Vorbehandlung mit Rhodanidionen sehr gering. Das zeigt, daß Fibrinogen bei Vorbehandlung der Probe mit Rhodanidionen nicht mitbestimmt wird.

**Tabelle 5**

| Abhängigkeit des Fibrinnachweises von der Rhodanid-Konzentration. | | | | |
|---|---|---|---|---|
| Probe | Absorption (mE/30 min) | | | |
| | Rhodanidionen-Konzentration in der Probenlösung (mol/l) | | | |
| | 1,5 | 3,0 | 4,0 | 5,0 |
| Standard 0 | 21 | 22 | 18 | 22 |
| Standard 1 µg/l | 61 | 67 | 72 | 69 |
| Standard 50 µg/ml | 1921 | 2009 | 2047 | 2055 |
| Normalplasma 3 | 28 | 43 | 43 | 46 |
| Normalplasma 4 | 27 | 62 | 75 | 74 |
| Path. Plasma 5 | 514 | 1083 | 1337 | 1376 |
| Path. Plasma 6 | 107 | 1230 | 1560 | 1569 |

Bei den verwendeten Rhodanidkonzentrationen ergeben sich keine Verfälschungen der Meßwerte beispielsweise durch Fibrinogen.

In einem weiteren Versuch konnte gezeigt werden, daß die Konzentration der Rhodanidionen bis auf 6,5 mol/l in der Probenlösung erhöht werden kann, ohne daß Meßwertverfälschungen entstehen.

**Tabelle 6**

| Abhängigkeit des Testsignals von der Dauer der Probeninkubation mit Rhodanid (4 mol/l Rhodanid in der Probenlösung). | | | | | |
|---|---|---|---|---|---|
| Probe | Absorption mE/30 min | | | | |
| | Inkubationsdauer (min) | | | | |
| | 1 | 3 | 5 | 10 | 30 |
| Fibrin Standard 50 µg/ml | 2228 | 2104 | 2038 | 2087 | 1942 |
| Path. Plasma Nr. 7 | 487 | 776 | 978 | 1094 | 1062 |
| Path. Plasma Nr. 8 | 312 | 687 | 514 | 1050 | 925 |
| Path. Plasma Nr. 9 | 658 | 1555 | 1257 | 1719 | 1552 |
| Path. Plasma Nr. 10 | 922 | 1159 | 1170 | 1257 | 1318 |

Die Bestimmung des Fibrins kann bereits kurze Zeit nach der Probenvorbehandlung, vorzugsweise 15 bis 30 min nach Zugabe der Rhodanid-Lösung erfolgen. Das zeigt auch Tabelle 7.

**Tabelle 7**

| Abhängigkeit des Meßsignals von der Dauer der Probenvorbehandlung (Rhodanid 4 mol/l in der Probenlösung). | | | |
|---|---|---|---|
| Probe | Inkubationsdauer (min) | Absorption (mE/30 min) | Fibrinkonzentration (gefunden) (µg/ml) |
| Normalplasma Nr. 5 | 15 | 112 | 0,8 |
| | 20 | 126 | 1,1 |
| | 28 | 125 | 1,1 |
| | 38 | 111 | 0,8 |
| | 51 | 111 | 0,8 |
| | 66 | 91 | 0,4 |
| Pathol. Plasma Nr. 11 | 21 | 1162 | 30,7 |
| | 26 | 1192 | 31,5 |
| | 34 | 1209 | 31,9 |
| | 44 | 1105 | 29,3 |
| | 57 | 1110 | 29,4 |
| | 72 | 1088 | 28,8 |
| Fibrin Standard (50 µg/ml) | | | |
| | 30 | 2075 | 50,0 |
| | 46 | 1877 | 46,4 |
| | 56 | 1955 | 47,8 |
| | 69 | 1968 | 48,1 |
| | 84 | 1688 | 42,6 |

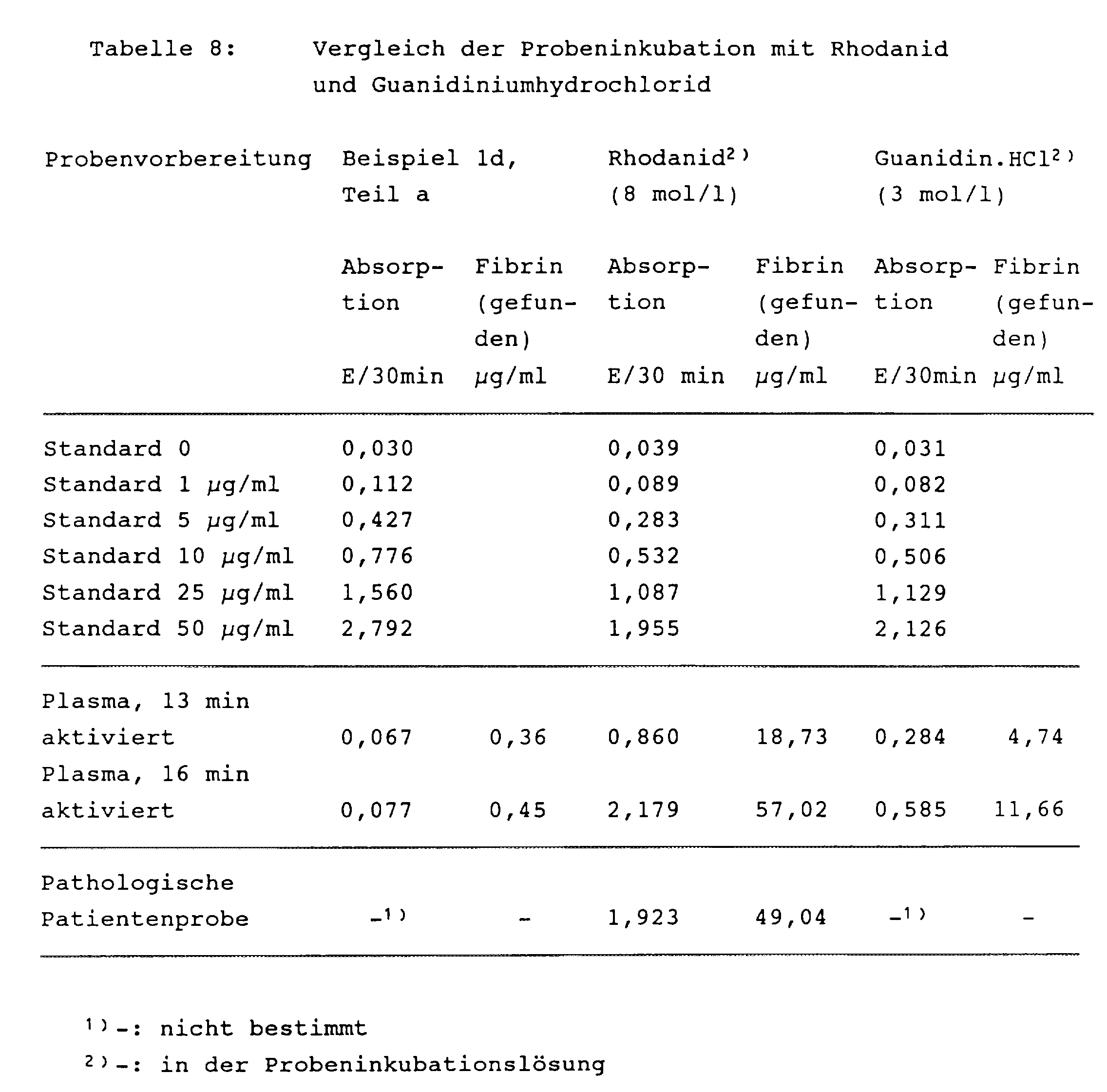

Die aktivierten Plasmaproben wurden analog dem Verfahren des in der Tabelle 3 beschriebenen Experiments durch Stehenlassen von Vollblut über einen Zeitraum von 13 bzw. 16 min gewonnen.

Das Experiment zeigt, daß durch Probenvorbereitung mit Guanidiniumhydrochlorid und Rhodanid eine Verbesserung des Fibrinnachweises erreicht wird.

In einem weiteren Experiment wurde die Wirkung des Zusatzes von Magnesiumionen auf die Sensitivität des Fibrinnachweises in Plasma untersucht. Die Ergebnisse zeigt Tabelle 9.

**Tabelle 9**

| Wirkung von Magnesiumnitrat auf die Sensitivität des Fibrin-Nachweises in Plasma. | | | | | | |
|---|---|---|---|---|---|---|
| Probe | Konzentration von Magnesiumnitrat in der Probeninkubationslösung | | | | | |
| | 1,0 mol/l | | 1,5 mol/l | | 2,0 mol/l | |
| | E/30 min | Fibrin µg/ml | E/30 min | Fibrin µg/ml | E/30min | Fibr µg/ml |
| Standard 0 | 0,031 | | 0,032 | | 0,031 | |
| Standard 1 | 0,100 | | 0,068 | | 0,070 | |
| Standard 5 | 0,381 | | 0,221 | | 0,207 | |
| Standard 10 | 0,747 | | 0,464 | | 0,420 | |
| Standard 25 | 1,569 | | 0,889 | | 0,855 | |
| Standard 50 | 2,823 | | 1,603 | | 1,689 | |
| Normalplasma | 0,042 | 0,17 | 0,040 | 0,29 | 0,038 | 0,20 |
| Normalplasma | 0,027 | 0,02 | 0,032 | 0,16 | 0,030 | 0,08 |
| Patholog.Plasma | 0,352 | 4,35 | 0,276 | 5,78 | 0,426 | 11,05 |

### Beispiel 3

Verbesserung des Fibrinnachweises durch Inkubation mit Jodid oder einer Kombination von Rhodanid und Jodid

Pathologische Plasmaproben, die nach der Vergleichsmethode nach Largo positiv reagieren, wurden, wie in Beispiel 1 d) angegeben, vorbehandelt. Die Bestimmung von Fibrin erfolgte, wie in Beispiel 2 angegeben.

Tabelle 10 zeigt die Wiederfindung von Fibrin nach Inkubation mit Jodid, Rhodanid und einer Kombination von Jodid und Rhodanid.

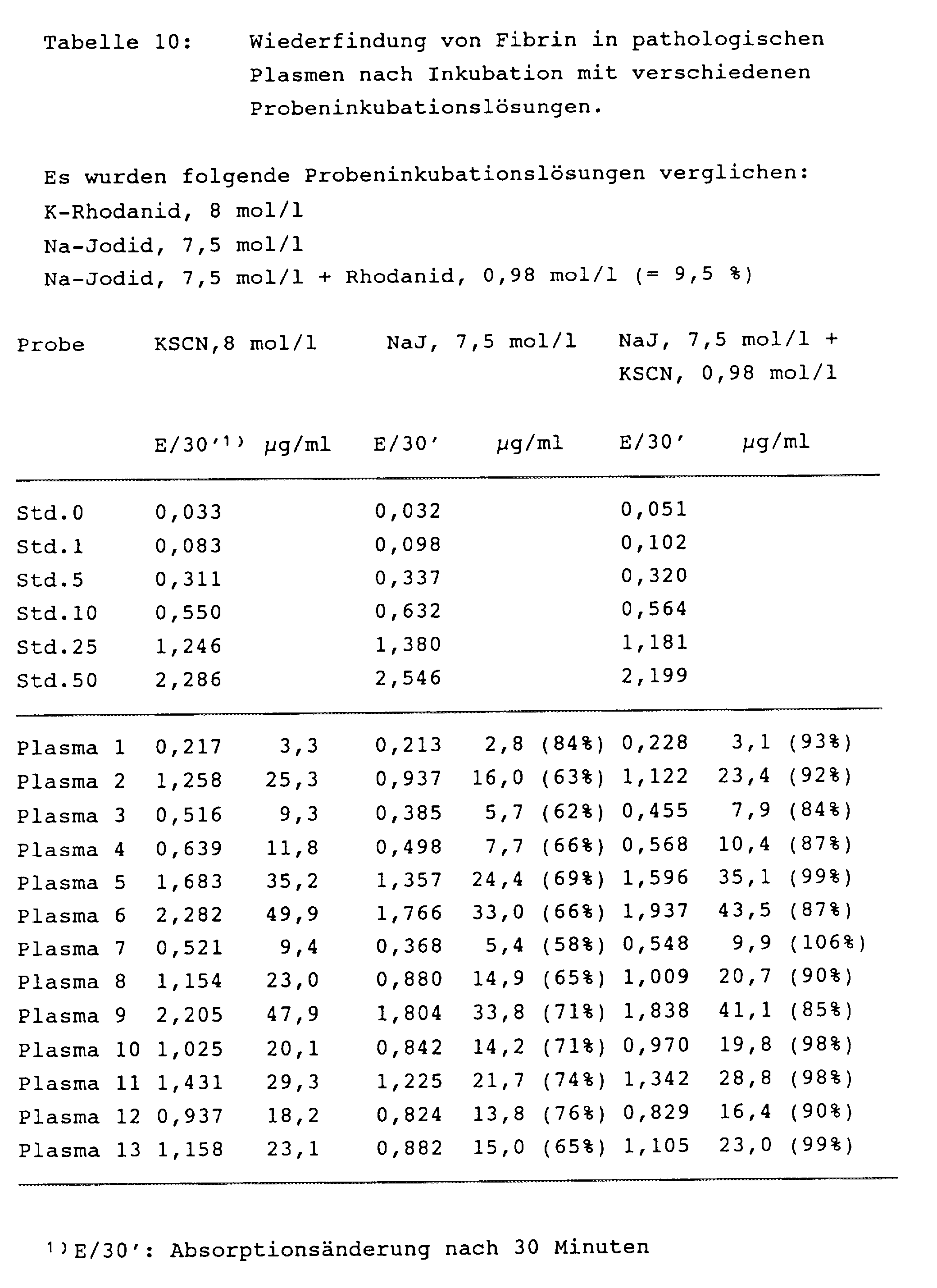

Tabelle 10 zeigt, daß die Kombination von Rhodanid und Jodid sich synergistisch ergänzt. Mit NaJ, 7,5 mol/l alleine wird diese Wirkung nur zu ca. 70 % erreicht. (Std. = Standard; - die Zahlen geben den Fibrinwert in µg/ml an). (Plasmen 1 bis 13: Die % - Zahlen in Klammern geben die Wiederfindung von Fibrin (µg/ml) an, bezogen auf Vorbehandlung der Probe mit Rhodanidionen, 8 mol/l).

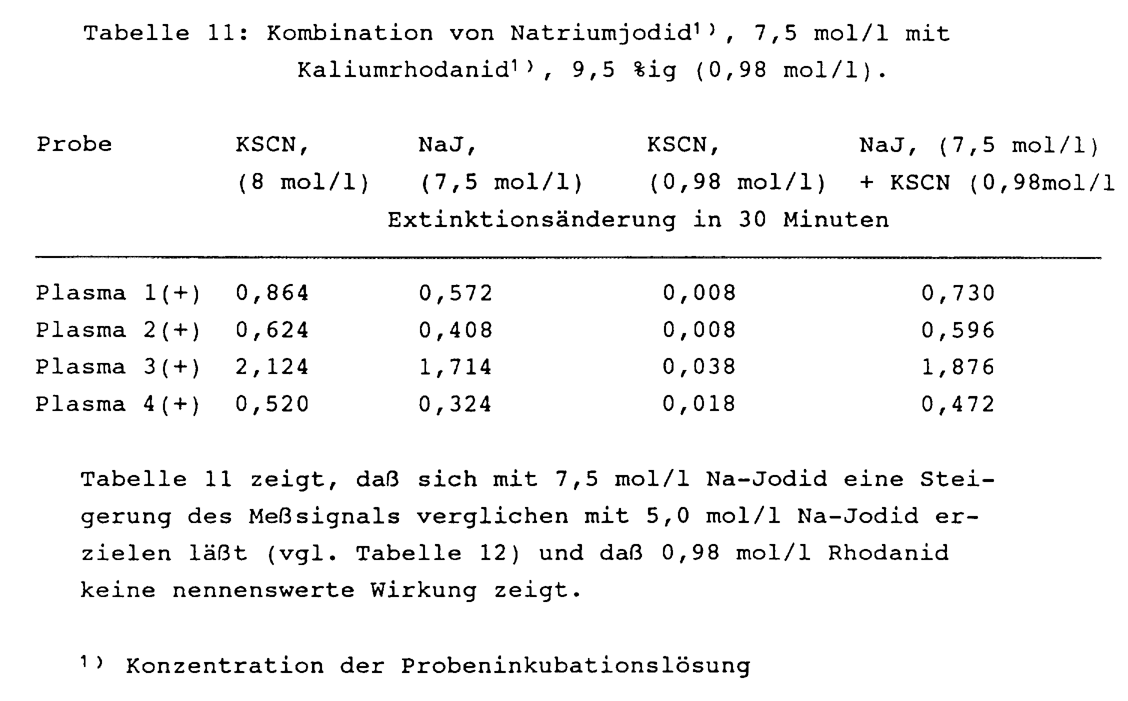

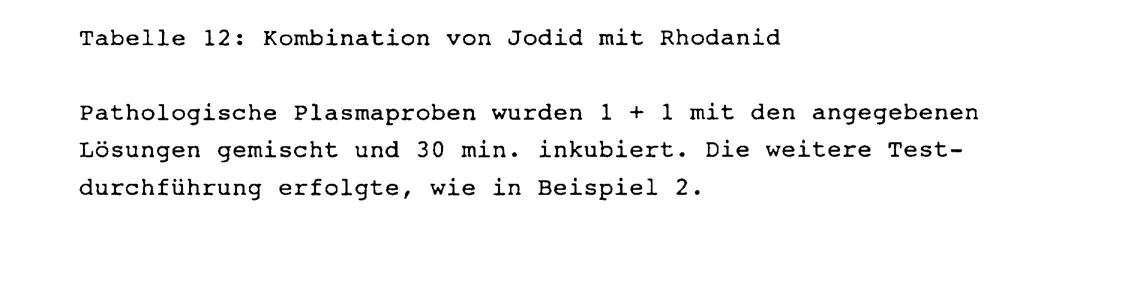

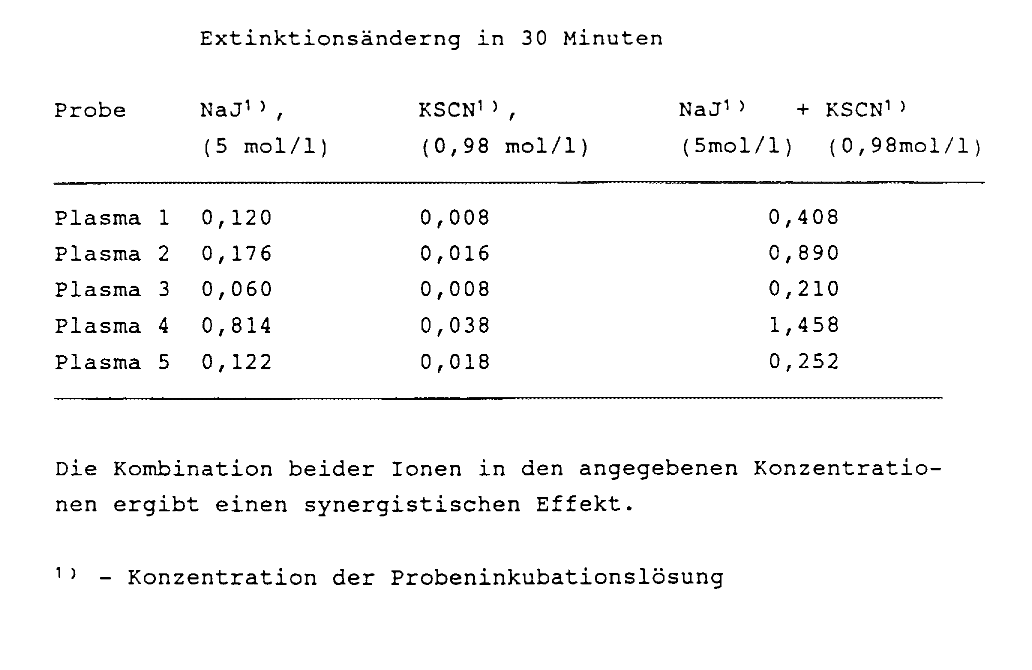

## Patentansprüche

1. Verfahren zur Behandlung einer Fibrin enthaltenden Probe
**dadurch gekennzeichnet**,
daß man die Probe mit Rhodanid-, Jodid-, Magnesiumoder/und Guanidiniumionen versetzt und die erhaltene Probenlösung inkubiert.

2. Verfahren nach Anspruch 1,
**dadurch gekennzeichnet,**
daß man die Probe mit Rhodanid- und Jodidionen inkubiert.

3. Methode zur Bestimmung von Fibrin in Körperflüssigkeiten,
**dadurch gekennzeichnet,**
daß man die Fibrin enthaltende Probe vor Durchführung der Bestimmung mit Rhodanid-, Jodid-, Magnesium-oder/und Guanidiniumionen versetzt, inkubiert, gegebenenfalls nach der Inkubation verdünnt und anschließend den Fibringehalt der Probenlösung ermittelt.

4. Methode nach Anspruch 3,
**dadurch gekennzeichnet,**
daß man nach der Inkubation den pH-Wert der Probenlösung auf 5,5 bis 7,5 einstellt.

5. Methode nach Anspruch 3 oder 4,
**dadurch gekennzeichnet,**
daß man nach der Inkubation die Probenlösung mit einem Puffer im Verhältnis von 1:5 bis 1:500 von Probenlösung zu Puffer verdünnt.

6. Methode nach einem der Ansprüche 3 bis 5,
**dadurch gekennzeichnet,**
daß man die Bestimmung von Fibrin unter Verwendung von mindestens einem fibrinspezifischen monoklonalen Antikörper durchführt.

7. Methode nach Anspruch 5,
**dadurch gekennzeichnet,**
daß man den Fibringehalt der Probenlösung durch einen heterogenen Sandwich-Assay ermittelt, wobei man die Probelösung mit einem immobilisierten oder immobilisierbaren fibrinspezifischen ersten Antikörper und einem markierten zweiten Antikörper, der an ein Konjugat von Fibrin und dem ersten Antikörper binden kann, in Kontakt bringt und die Markierung auf an sich bekannte Weise bestimmt.

8. Methode nach Anspruch 7,
**dadurch gekennzeichnet,**
daß man als ersten Antikörper einen mit einer Streptavidin-beschichteten Festphase bindefähigen biotinylierten Antikörper oder ein biotinyliertes Antikörperfragment verwendet.

9. Methode nach Anspruch 7 oder 8,
**dadurch gekennzeichnet,**
daß man als zweiten Antikörper einen Antikörper oder ein Antikörperfragment mit einer enzymatischen Markierungsgruppe verwendet.

10. Reagenzienkit zur Bestimmung von Fibrin in Körperflüssigkeiten,
**dadurch gekennzeichnet,**
daß er Rhodanid, Jodid, Magnesium oder/und Guanidiumsalze in fester oder/und gelöster Form sowie Reagenzien für die Fibrinbestimmung enthält.

## Claims

1. Process for the treatment of a sample containing fibrin,
**characterised in that** thiocyanate, iodide, magnesium or/and guanidinium ions are added to the sample and the sample solution obtained is incubated.

2. Process according to claim 1,
**characterised in that** the sample is incubated with thiocyanate and iodide ions.

3. Method for determining fibrin in body liquids,
**characterised in that** thiocyanate, iodide, magnesium and/or guanidinium ions are added to the sample containing fibrin prior to carrying out determination, followed by incubation, if desired dilution after incubation and subsequently determination of the content of fibrin in the sample solution.

4. Method according to claim 3,
**characterised in that** after incubation the pH value of the sample solution is adjusted to 5.5 to 7.5.

5. Method according to claim 3 or 4,
**characterised in that** after incubation the sample solution is diluted with a buffer at a ratio of from 1:5 to 1:500 of sample solution to buffer.

6. Method according to one of claims 3 to 5,
**characterised in that** determination of fibrin is carried out using at least one monoclonal antibody specific to fibrin.

7. Method according to claim 5,
**characterised in that** the fibrin content of the sample solution is determined by a heterogeneous sandwich assay, wherein the sample solution is contacted with an immobilized or immobilizable first antibody specific to fibrin and a labelled second antibody capable of binding to a conjugate of fibrin and the first antibody and wherein the label is determined in a manner which is per se known.

8. Method according to claim 7,
**characterised in that** a biotinylated antibody capable of binding to a streptavidin-coated solid phase or a biotinylated antibody fragement is used as the first antibody.

9. Method according to claim 7 or 8,
**characterised in that** an antibody or an antibody fragment with an enzymatic marker group is used as the second antibody.

10. Reagent kit for determining fibrin in body fluids,
**characterised in that** it contains thiocyanate, magnesion or/and guanidinium salts in a solid and/or dissolved form as well as reagents for the determination of fibrin.

## Revendications

1. Procédé de traitement d'un échantillon contenant de la fibrine, caractérisé en ce que l'on ajoute à l'échantillon des ions thiocyanate, iodure, magnésium et/ou guanidinium et on met à incuber la solution d'échantillon obtenue.

2. Procédé selon la revendication 1, caractérisé en ce que l'on met l'échantillon à incuber avec des ions tbiocyanate et iodure.

3. Procédé de dosage de la fibrine dans des liquides corporels, caractérisé en ce que, avant d'effectuer le dosage, on ajoute des ions thiocyanate, iodure, magnésium et/ou guanidinium à l'échantillon contenant la fibrine, on le met à incuber, on le dilue éventuellement après l'incubation, puis on détermine la teneur en fibrine de la solution d'échantillon.

4. Procédé selon la revendication 3, caractérisé en ce que, après l'incubation, on ajuste le pH de la solution d'échantillon à une valeur de 5,5 à 7,5.

5. Procédé selon la revendication 3 ou 4, caractérisé en ce que, après l'incubation, on dilue la solution d'échantillon avec un tampon à un rapport de la solution d'échantillon au tampon compris entre 1:5 et 1:500.

6. Procédé selon l'une des revendications 3 à 5, caractérisé en ce que l'on effectue le dosage de la fibrine à l'aide d'au moins un anticorps monoclonal spécifique de la fibrine.

7. Procédé selon la revendication 5, caractérisé en ce que l'on détermine la teneur en fibrine de la solution d'échantillon au moyen d'une analyse en sandwich hétérogène, en mettant en contact la solution d'échantillon avec un premier anticorps spécifique de la fibrine immobilisé ou immobilisable et un second anticorps marqué capable de se lier à un conjugué de fibrine et du premier anticorps, et en dosant de façon connue le marqueur.

8. Procédé selon la revendication 7, caractérisé en ce que l'on utilise comme premier anticorps un anticorps biotinylé ou un fragment d'anticorps biotinylé capable de se lier à une phase solide revêtue de streptavidine.

9. Procédé selon la revendication 7 ou 8, caractérisé en ce que l'on utilise comme second anticorps un anticorps ou un fragment d'anticorps comportant un groupe marqueur enzymatique.

10. Trousse de réactifs pour le dosage de la fibrine dans des liquides corporels, caractérisée en ce qu'elle contient des sels d'ions thiocyanate, iodure, magnésium et/ou guanidinium sous forme solide et/ou dissoute, et des réactifs pour le dosage de la fibrine.
